# FASCICULE DE BREVET EUROPEEN

(11) **EP 2 146 569 B1**
(45) Date de publication et mention de la délivrance du brevet: **01.06.2011**
(21) Numéro de dépôt: 08787815.3
(22) Date de dépôt: 19.03.2008
(51) Int. Cl.: A01K 67/033

(54) **OBTENTION DE MOLLUSQUES BIVALVES TETRAPLOÏDES A PARTIR DE GENITEURS DIPLOÏDES**
HERSTELLUNG TETRAPLOIDER MUSCHELMOLLUSKEN AUS DIPLOIDEN ELTERN
PRODUCTION OF BIVALVE TETRAPLOID MOLLUSCS FROM DIPLOID PARENTS

(30) Priorité: 23.03.2007 FR 0702123
(43) Date de publication de la demande: 27.01.2010
(73) Titulaire: INSTITUT FRANCAIS DE LA RECHERCHE POUR L'EXPLOITATION DE LA MER (IFREMER), 92130 Issy Les Moulineaux (FR)
(72) Inventeur: BENABDELMOUNA, Abdellah, F-17300 Rochefort (FR); LEDU, Christophe, F-17390 La Tremblade (FR)
(74) Mandataire: Vialle-Presles, Marie José
(86) Numéro de dépôt international: PCT/FR2008/000362
(87) Numéro de publication internationale: WO 2008/132350

(56) Documents cités:
- WO-A-95/19703
- GUO X ET AL: "GENETIC CONSEQUENCES OF BLOCKING POLAR BODY I WITH CYTOCHALASIN B IN FERTILIZED EGGS OF THE PACIFIC OYSTER, CRASSOSTREA GIGAS: I. PLOIDY OF RESULTANT EMBRYOS" BIOLOGICAL BULLETIN, LANCASTER PRESS, INC., LANCASTER, PA, US, vol. 183, no. 3, 1 décembre 1992 (1992-12-01), pages 381-386, XP000613265 ISSN: 0006-3185 cité dans la demande
- SCARPA JOHN ET AL: "Induction of tetraploidy in mussels by suppression of polar body formation" NIPPON SUISAN GAKKAISHI - BULLETIN OF THE JAPANESE SOCIETY OF SCIENTIFIC FISHERIES, NIPPON SUISAN GAKKAI, TOKYO, JP, vol. 59, no. 12, 1993, pages 2017-2023, XP009090921 ISSN: 0021-5392
- PERUZZI STEFANO ET AL: "Tetraploid induction by meiosis inhibition with cytochalasin B in the dwarf surfclam, Mulinia lateralis say: Effects of temperature" JOURNAL OF SHELLFISH RESEARCH, NATIONAL SHELLFISHERIES ASSOCIATION, US, vol. 21, no. 2, décembre 2002 (2002-12), pages 677-684, XP009090924 ISSN: 0730-8000
- GUO X ET AL: "VIABLE TETRAPLOIDS IN THE PACIFIC OYSTER (CRASSOSTREA GIGAS THUNBERG) PRODUCED BY INHIBITING POLAR BODY 1 IN EGGS FROM TRIPLOIDS" MOLECULAR MARINE BIOLOGY AND BIOTECHNOLOGY, vol. 3, no. 1, 1 février 1994 (1994-02-01), pages 42-50, XP000613251 cité dans la demande

## Description

La présente invention est relative à la production de mollusques bivalves tétraploïdes viables.

La production de bivalves polyploïdes fait actuellement l'objet d'un grand intérêt, en particulier en ce qui concerne les huîtres.

En effet, les huîtres triploïdes, également dénommées « huîtres des 4 saisons » présentent plusieurs avantages. Du fait de leur nombre impair de chromosomes, elles sont normalement stériles, ce qui leur permet d'une part d'avoir une croissance plus rapide (leur énergie étant utilisée pour la croissance plutôt que pour la reproduction), et d'autre part de ne pas produire de laitance pendant les mois d'été, ce qui leur assure une qualité constante toute l'année.

Des huîtres triploïdes peuvent être obtenues par croisement de géniteurs diploïdes, suivi de la rétention du premier ou du second globule polaire par traitement chimique, généralement par la cytochalasine B (CB) ou éventuellement par le 6-diméthyl-aminopurine (6-DMAP), ou par traitement physique (notamment choc thermique ou de pression). Ces deux techniques présentent toutefois un certain nombre d'inconvénients. On citera notamment un manque d'homogénéité du niveau de ploïdie ; en effet, les traitements induisant la rétention des globules polaires aboutissent à des populations larvaires mélangées, possédant des niveaux de ploïdie variés. A la fin du développement larvaire, la population obtenue contient généralement au maximum 90% d'individus triploïdes, le reste de la population étant constitué de diploïdes. En outre, dans le cas des traitements chimiques, ceux-ci, qui utilisent des produits très toxiques, sont mal perçus du public, et nécessitent de mettre constamment en oeuvre des précautions importantes pour éviter tout danger pour les manipulateurs.

C'est pourquoi la méthode actuellement privilégiée pour la production d'huîtres triploïdes consiste à croiser des huîtres tétraploïdes avec des huîtres diploïdes. Cette méthode présente, outre l'avantage de ne pas nécessiter de recours répété à des traitements chimiques, celui d'aboutir à une population homogène dont la totalité des individus sont triploides. Le principal inconvénient de cette méthode provient de la difficulté d'obtenir des géniteurs tétraploïdes viables.

La production de larves tétraploïdes, en proportions variables, a été observée au cours d'expérimentations de production de triploïdes par croisement de géniteurs diploïdes et rétention de l'expulsion du premier globule polaire par traitement à la cytochalasine B (CB). Toutefois, cette production de larves tétraploïdes apparaît transitoire ; elle n'a été décrite que durant les premières 24 heures post-fécondation (STEPHENS & DOWNING, J. Shellfish Res, 7, 550-51, 1988; GUO et al., Biol. Bull., 183, 381-93, 1992). Les larves tétraploïdes détectées voient par la suite leur proportion chuter drastiquement, de telle façon qu'aucune larve tétraploïde n'est détectée ni durant le reste de l'élevage larvaire, ni au moment de la fixation et à fortiori lors des élevages en micronurserie ou en nurserie.

Deux hypothèses ont été émises afin d'expliquer l'échec de toutes les approches utilisées afin d'obtenir des tétraploïdes viables directement à partir de géniteurs diploïdes :
Selon la première de ces hypothèses, la tétraploïdie entraînerait un fardeau génétique incompatible avec la survie des larves du fait de l'expression de certains allèles létaux récessifs dont le nombre de copies se retrouve changé suite à la tétraploïdisation (hypothèse des allèles létaux récessifs). Toutefois, des expériences de gynogenèse utilisant des lignées consanguines et sauvages tendent à montrer que l'expression des allèles létaux récessifs ne peut pas expliquer à elle seule la totalité de la mortalité des tétraploïdes durant le stade larvaire (GUO, Ph.D. Dissertation : Studies on tetraploid induction in the Pacific oyster, Crassostrea gigas., University of Washington, Seattle 1991).

Selon la seconde de ces hypothèses (hypothèse du rapport nucléo-cytoplasmique), les ovocytes produits par les géniteurs diploïdes femelles possèdent une taille trop faible pour supporter un noyau dont le matériel génétique se trouve doublé suite à la tétraploïdisation. C'est cette seconde hypothèse qui est a été privilégiée, et c'est sur cette base qu'ont été développées les méthodes proposées jusqu'à présent pour obtenir des huîtres tétraploïdes viables.

La première méthode à avoir été mise en oeuvre avec succès consiste à croiser des géniteurs femelles triploïdes avec des géniteurs mâles diploïdes, et à bloquer la première phase de la méiose (MI) en induisant la rétention du premier globule polaire (Demande PCT WO 95/19703; GUO & ALLEN, Mol Mar Biol Biotechnol 3, 42-50, 1994). Cette méthode est basée sur le fait que les quelques rares femelles triploïdes qui ne sont pas stériles, produisent des ovocytes dont la taille est bien supérieure à celle des ovocytes produits par les femelles diploïdes, ce qui leur permet de supporter un noyau tétraploïde.

Récemment, la production d'huîtres tétraploïdes viables à partir d'un croisement de mâles tétraploïdes avec des femelles diploïdes, préalablement sélectionnées pour la grande taille de leurs ovocytes, suivi de la rétention du second globule polaire a également été décrite (MCCOMBIE et al., Ma Biotechnol (NY), 7, 318-30, 2005). Cependant, à l'heure actuelle, la seule méthode de production de tétraploïdes utilisée en pratique, est celle mettant en oeuvre le croisement de femelles triploïdes avec des mâles diploïdes. Cette méthode présente toutefois deux inconvénients majeurs :
1) Ces tétraploïdes étant obtenus à partir de triploïdes qui présentent une fertilité non négligeable, l'utilisation de ces mêmes tétraploïdes afin de produire de nouvelles générations de triploïdes peut entrainer une augmentation de la fertilité des triploïdes issus des générations successives. Ainsi, il a été observé que la fertilité moyenne passait de 2% pour les triploïdes de premiere génération (triploïdes « chimiques » obtenus après rétention du second GP suite à un traitement à la CB) à 13,4% pour les triploïdes de seconde génération, issus d'un croisement de géniteurs mâles tétraploïdes avec des femelles diploïdes (GUO & ALLEN, Biol. Bull., 187, 309-18, 1994). Cette augmentation de fertilité au cours des générations risque d'entraîner l'apparition à plus ou moins long terme d'une population de triploïdes dont la fertilité irait croissant, ce qui fait courir un risque d'une stérilisation progressive du milieu, et de contamination des stocks d'huîtres diploïdes autochtones suite à la reproduction des huîtres triploïdes dont la descendance est majoritairement aneuploïde.
2) La nécessité de passer par des triploïdes rend particulièrement difficile et fastidieuse l'introduction, au niveau tétraploïde, de tout progrès génétique normalement et généralement réalisé au niveau diploïde. En effet, les lignées diploïdes améliorées suite à des programmes de sélection génétique doivent d'abord être utilisées pour produire des triploïdes par des méthodes classiques d'induction chimique. Ce n'est qu'ensuite que les quelques triploïdes qui arrivent à entrer en maturation et à produire des ovocytes peuvent être utilisés comme parents femelles afin de produire des tétraploïdes.

Il apparaît donc particulièrement souhaitable de disposer d'une méthode de production d'huîtres tétraploïdes qui permette le passage direct du stade diploïde au stade tétraploïde sans passer par le stade triploïde.

Les Inventeurs ont émis l'hypothèse que la non-survie, au delà du stade larvaire, des tétraploïdes produits lors du croisement de géniteurs diploïdes pouvait avoir d'autres causes que la taille trop faible de l'ovocyte diploïde par rapport au noyau tétraploïde, et que parmi ces causes pouvait notamment figurer une baisse de la valeur adaptative générale (c'est-à-dire de l'aptitude globale à la survie) résultant de la tétraploïdie. Cette baisse de la valeur adaptative pourrait être due à l'expression de certains allèles négatifs qui se trouvent normalement réprimés chez les diploïdes et les triploïdes et ne seraient pas réprimés, ou seulement partiellement réprimés chez les tétraploïdes.

Elle aurait pour résultat une faible compétitivité des larves tétraploïdes, et donc leur disparition rapide lorsqu'elles sont élevées en mélange avec des larves diploïdes et triploïdes, qui sont également présentes dans les populations larvaires résultant du croisement de géniteurs diploïdes suivi de la rétention du premier globule polaire.

A partir de cette hypothèse, les Inventeurs ont postulé que le tri des larves, afin d'isoler les tétraploïdes et de leur permettre de s'affranchir de la très forte compétition exercée par les larves diploïdes et triploïdes permettrait d'aboutir à l'obtention de tétraploïdes viables, à partir d'un croisement impliquant uniquement des géniteurs diploïdes sauvages.

Ils ont donc développé une méthode basée sur la détection des larves tétraploïdes, l'isolement d'une sous-population larvaire contenant un fort pourcentage de larves tétraploïdes, et l'élevage de cette sous-population, ces étapes étant répétées autant de fois que nécessaire au cours du développement.

Ils ont ainsi constaté que les larves tétraploïdes se distinguaient notamment par un développement moins rapide et donc une taille plus faible que celle des larves diploïdes et triploïdes au même stade de développement. Cette observation confirme l'hypothèse formulée par les Inventeurs, de la baisse de la valeur adaptative des larves tétraploïdes. Elle permet en outre de simplifier le tri des larves pour l'obtention de populations enrichies en larves tétraploïdes, puisque ce tri peut être effectué sur la base de la taille larvaire.

La présente invention a en conséquence pour objet un procédé d'obtention de bivalves tétraploïdes, caractérisé en ce qu'il comprend :
a) la fécondation d'ovocytes de femelles diploïdes par du sperme de males diploïdes, suivie de l'induction de la rétention du premier corps polaire des oeufs fécondés ;
b) la culture des larves obtenues à l'issue de cette fécondation ;
c) l'isolement, à partir de la population de larves cultivées à l'étape b), d'une sous-population enrichie en tétraploïdes, comprenant au moins 20%, de préférence au moins 30% de larves tétraploïdes;
d) la culture de la sous-population de larves isolée à l'étape c).

La rétention du premier corps polaire peut être induite, de manière classique, par tout traitement induisant l'inhibition de l'assemblage des microtubules, généralement effectué entre la cinquième et la vingt-cinquième minute post-fécondation, et dont la durée peut varier de 10 à 20 minutes. Il peut s'agir par exemple d'un traitement par un agent chimique, tel que colchicine, caféine, nocodazole, cytochalasine B, ou 6-DMAP, ou d'un traitement physique tel qu'un choc thermique modéré ou un choc hyperbare.

Selon un mode de mise en oeuvre préféré de la présente invention, la rétention du premier corps polaire est induite par traitement à la cytochalasine B. Le traitement à la cytochalasine B peut être effectué entre la 5^{ème} et la 25^{ème} minute après la fécondation, pendant 10 à 20 minutes. De préférence il est effectué à partir de la 10^{ème} minute après la fécondation, pendant environ 15 minutes. De manière particulièrement avantageuse, la cytochalasine B est utilisée à une concentration finale par litre de milieu de traitement comprise entre 0,3 et 0,7 mg, de préférence de l'ordre de 0,5 mg. Ces concentrations, qui sont deux fois plus faibles que celles habituellement utilisées pour l'induction de la rétention du premier corps polaire dans le cadre de la production d'huîtres triploides, permettent de limiter l'effet toxique de la cytochalasine B, sans toutefois diminuer l'efficacité de l'induction.

On peut également utiliser, à la place de la cytochalasine B, un autre agent chimique, tel que colchicine, caféine, nocodazole, ou 6-DMAP (300 à 500 µM), ou un traitement physique tel qu'un choc thermique modéré (30 à 40°C) ou un choc hyperbare. Pour chacun de ces traitements, les conditions choisies seront de préférence plus modérées que lorsque le même traitement est mis en oeuvre pour induire la rétention du premier corps polaire dans le cadre de la production d'huîtres triploïdes.

De manière particulièrement avantageuse, le procédé conforme à l'invention comprend les étapes supplémentaires suivantes:
e) l'isolement, à partir de la sous-population de larves cultivées à l'étape précédente, d'une sous-population riche en tétraploïdes comprenant au moins 20%, avantageusement au moins 30%, de larves tétraploïdes ;
f) la culture de la sous-population de larves isolée à l'étape e).

Généralement, la culture de l'étape b) est effectuée pendant 6 à 10 jours, de préférence pendant environ 8 jours, et la culture des étapes d) et f) est effectuée pendant 2 à 3 jours.

Ces étapes d'isolement d'une sous-population riche en tétraploïdes et de culture de la sous-population isolée peuvent être répétées plusieurs fois, jusqu'à ce que les larves aient atteint le stade pédivéligère (taille comprise entre 220 et 250 µm). Avantageusement elles sont répétées au moins 3 fois, de préférence au moins 4 fois, à des intervalles de 2 à 3 jours.

Selon un mode de mise en oeuvre particulièrement préféré du procédé conforme à l'invention, le tri des larves pour isoler une sous-population enrichie en tétraploïdes, est effectué sur la base de leur taille.

En effet, comme indiqué ci-dessus, les larves tétraploïdes ont une taille plus réduite que les larves diploïdes et triploïdes au même stade de développement. Généralement, la taille moyenne des larves tétraploïdes est inférieure de 20 à 30% à celle des larves diploïdes, et de 25 à 35% à celle des larves triploïdes de la même espèce au même stade de développement, et cultivées dans les mêmes conditions.

Ceci permet de trier facilement les larves, par simple passage à travers un tamis ayant une taille de maille appropriée, choisie en fonction de l'espèce concernée, du stade de développement larvaire, et des conditions de culture.

De manière avantageuse, des étapes supplémentaires d'isolement d'une sous-population enrichie en tétraploïdes et de culture de la sous-population isolée peuvent être effectuées après le stade pédivéligère, sur les naissains fixés. A partir de ce stade, elles seront de préférence répétées à des intervalles moins rapprochés que précédemment, par exemple tous les 8 à 15 jours.

Dans ce cas, pour effectuer le tri par tamisage, le support de fixation fourni aux larves pédivéligères est constitué de particules de taille à peu près égale à celle des larves lors de la fixation, ce qui permet la fixation d'une seule larve par particule. On peut notamment utiliser comme support des particules de microbrisure, obtenue par broyage de coquilles d'huîtres.

Après chacune des étapes d'isolement d'une sous-population, le pourcentage de tétraploïdes dans la sous-population sélectionnée peut, si on le souhaite, être vérifié par des méthodes classiques de détermination de niveau de ploïdie. On peut par exemple utiliser la cytométrie de flux, sur un échantillon, d'au moins 100 individus, prélevé à partir de ladite sous-population.

Le procédé conforme à l'invention peut être utilisé pour toutes les espèces de bivalves (par exemple pectinidés, moules *(Mytilus edulis* et *Mytilus galloprovincialis),* palourdes, etc.) chez lesquelles on désire produire des tétraploïdes (notamment en vue de les utiliser pour la production de triploïdes). Il est tout particulièrement intéressant pour la production d'huîtres tétraploïdes, et peut par exemple être utilisé, non seulement chez des huîtres de l'espèce *Crassostrea gigas,* comme illustré par les exemples ci-après, mais également chez toute autre espèce du genre *Crassostrea* (notamment *Crassostrea virginica), Saccostrea* (par exemple *Saccostrea commerciales),* ou chez des espèces du genre *Ostrea,* telles qu'*Ostrea edulis.*

Le procédé conforme à l'invention permet d'obtenir des bivalves tétraploïdes viables, et notamment des huîtres, à partir de n'importe quels géniteurs diploïdes. En particulier, il ne nécessite aucune sélection des géniteurs femelles en ce qui concerne la taille de leurs ovocytes.

Il permet de produire des géniteurs tétraploïdes, directement à partir de parents sauvages diploïdes et ceci sans passer par une étape triploïde. Ces géniteurs tétraploïdes peuvent ensuite être utilisés pour la production de triploïdes sans encourir les risques, mentionnés ci-dessus, liés à une augmentation de la fertilité des triploïdes au cours des générations.

Le procédé conforme à l'invention permet également d'introduire directement chez des tétraploïdes, des améliorations génétiques obtenues chez des diploïdes. Il suffit en effet pour cela d'utiliser comme matériel de départ pour la mise en oeuvre de ce procédé, des ovocytes et du sperme obtenus à partir d'animaux d'une lignée diploïde sélectionnée. Les tétraploïdes obtenus peuvent ensuite être utilisés pour l'obtention de triploïdes intégrant également la même amélioration.

La présente invention sera mieux comprise à l'aide du complément de description qui va suivre, qui se réfère à des exemples illustrant la mise en oeuvre de la présente invention pour l'obtention d'huîtres tétraploïdes viables.

### EXEMPLE 1 : INDUCTION DE LA POLYPLOÏDIE ET ELEVAGE LARVAIRE

Les géniteurs diploïdes utilisés pour les expérimentations proviennent d'un captage naturel dans le bassin de Marennes Oléron de naissain diploïde sauvage. Six géniteurs femelles et quatre géniteurs mâles ont été utilisés pour la récolte des gamètes par scarification des gonades. 15 millions d'ovocytes et 3 milliards de spermatozoïdes issus des dix géniteurs ont été utilisés pour réaliser le croisement dans 1 l d'eau de mer filtrée à 25°C. La rétention du premier globule polaire a été réalisée en utilisant la cytochalasine B (CB), dissoute dans le DMSO, et à la concentration finale de 0,5 mg/l. La CB a été administrée pendant 15 minutes et ceci à partir de la dixième minute après la fécondation.

Ce protocole d'induction de polyploïdie est similaire à ceux déjà publiés dans la littérature dans le cadre de l'obtention de triploïdes (GUO et al., Biol. Bull., 183, 381-93, 1992 ; GÉRARD et al., Aquaculture, 174, 229-42 1999). Cependant, la concentration finale de CB a été réduite (0,5 mg/l au lieu de 1 mg/l) , afin de limiter l'effet toxique de ce produit sans toutefois diminuer le taux d'induction de polyploïdie.

La CB est ensuite éliminée après tamisage des embryons au travers d'un filtre nylon de 10 µm de taille de mailles et un lavage de 15 minutes dans de l'eau de mer filtrée contenant 0,1% DMSO.

A titre de témoin, 15 millions d'ovocytes et 5 milliards de spermatozoïdes issus des mêmes géniteurs ont été utilisés pour réaliser un croisement dans les mêmes conditions, à l'exception du traitement à la CB.

Ensuite, les embryons de chacun des deux groupes (groupe témoin et groupe traité à la CB) sont remis en culture dans des bacs cylindro-coniques contenant 150 1 d'eau de mer filtrée à 22°C et aérée grâce à un bulleur incorporé dans le bac d'élevage.

Afin de pouvoir estimer les taux d'éclosion et une éventuelle mortalité des larves, ces dernières sont tamisées après 24 heures post-fécondation, au travers d'un filtre de 45 µm de diamètre, reprises dans une éprouvette graduée contenant 1 ou 2 l d'eau de mer filtrée et le nombre total de larves est estimé à partir d'un comptage dans un échantillon de 50 µl.

D'autre part, la ploïdie des larves est déterminée par cytométrie en flux. Après chaque tamisage, un échantillon de larves (100 larves au minimum) est prélevé et mis dans un tube de 1,5 ml. Après élimination de l'eau de mer par une brève centrifugation, les larves sont reprises dans 1 ml de tampon de lyse (5 mM MgCl₂, 85 mM NaCl, 10 mM Tris, 0,1% Triton X-10C, pH7). Ensuite, les larves sont broyées à l'aide d'un piston en plastique et les noyaux libérés sont filtrés au travers d'un filtre en nylon de 30 µm de taille de maille (Partec Celltrics) et recueillis dans un tube d'analyse de cytométrie, puis marqués au DAPI (4',6-Diamidino-2-phenylindole dihydrochloride) à la concentration finale de 1 µg/ml, par addition de 1 ml de tampon de lyse contenant le DAPI à 2 µg/nl et 2 µl de témoin interne de ploïdie (érythrocytes de truite, TRBC, Coulter DNA Reference Calibrator, 629972).

Après une incubation minimale de 30 min sur glace et à l'obscurité, les analyses cytométriques sont réalisées en utilisant un cytomètre Partec PAII. Chaque analyse (canal FL4, échelle linéaire de 1024 canaux) se fait sur un minimum de 2000 particules. Les résultats obtenus sont présentés sous forme d'histogrammes de fréquences avec une distribution gaussienne des événements au sein de chaque pic. Les pics sont noté « peak x » quand leur identification a été réalisée de façon automatique par le logiciel FloMax^{™}, ou « RN/PK x » quand leur identification a été réalisée manuellement par l'opérateur (x étant le numéro du pic).

Dans le groupe traité à la CB, le taux de larves D à J1 est estimé à 16,5% (contre 42% pour le témoin non traité) ce qui représente une population de larve D d'à peu près 2,5 millions de larves.

A partir du deuxième jour post-fécondation, le renouvellement d'eau se fait tous les deux jours après tamisage. Jusqu'à J6 post-fécondation, les larves ont été tamisées au travers d'un filtre de 45 µm. A chaque renouvellement, les larves sont comptées et leur niveau de ploïdie déterminé par cytométrie de flux, comme décrit ci-dessus.

Ensuite, les larves sont remises dans leurs cuves d'élevage cylindroconiques respectives en respectant une densité maximale de 10000 larves/l à J4 post-fécondation et de 6500 larves/l à J6 post-fécondation.

Les larves sont nourries tous les jours par un mélange d'algues fourrage cultivées dans le laboratoire et contenant 70% de *Isochrysis galbana* souche Tahiti et 30 % de *Chaetoceros gracilis à* la concentration de 25 cellules/µl/jour.

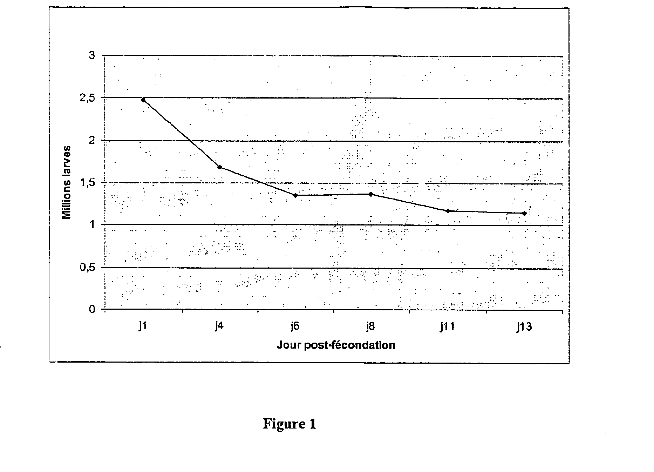

Le suivi du taux de survie au cours des premiers jours de l'élevage montre une nette diminution de la survie des larves entre J1 et J6 post-fécondation (Figure 1). Cette diminution peut s'expliquer à la fois par l'effet nocif du traitement à la CB et surtout par l'effet de la forte densité de larves dans le bac d'élevage.

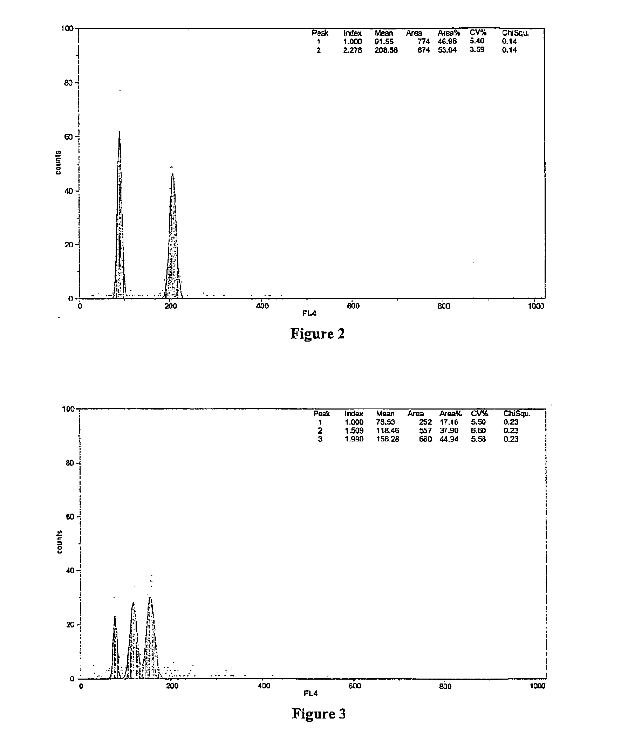

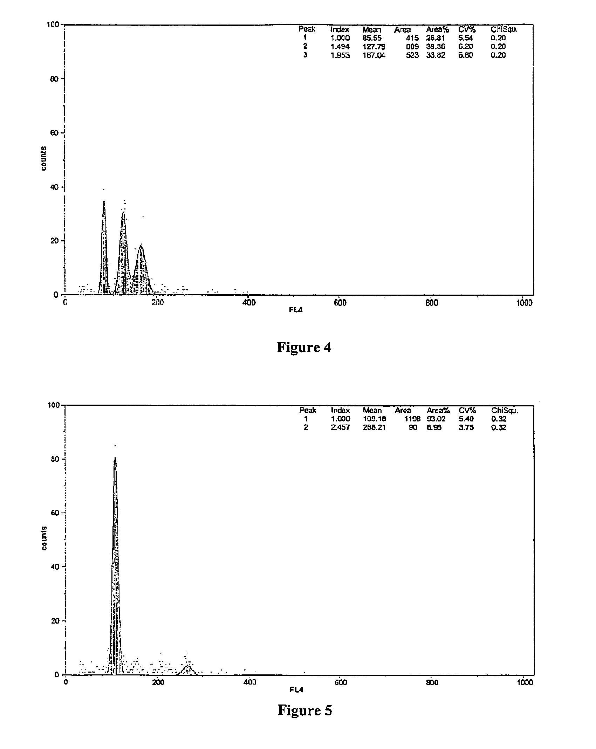

Entre J1 et J6 post-fécondation, les analyses cytometriques réalisées à partir des larves traitées ou non à la CB montrent que :
- Le croisement témoin présente une population de noyaux exclusivement diploïde (Figure 2, « peak 1 »),
- Les échantillons de larves issus de l'induction de rétention de GPI montrent (Figures 3 et 4) trois types de populations nucléaires correspondant aux niveaux de ploïdie suivants : diploïde (autour de 20% en moyenne, « peak 1 »), triploïde (autour de 40 % en moyenne, « peak 2 ») et tétraploïde (autour de 40% en moyenne, « peak 3 »).

Après J6 post-fécondation, la population de larves se stabilise et l'élevage larvaire continue sans mortalité notable jusqu'à fixation des larves.

Les tamisages sont réalisés le matin et tous les deux ou trois jours. Jusqu'au sixième jour post fertilisation, les larves ont été tamisées, de manière non sélective, au travers d'un tamis de 45 µm.

Par la suite, des tamisages sélectifs ont été effectués, en adaptant la taille des tamis à la population de larves ciblée, et différentes populations de larves ont été constituées, en se basant sur leur vitesse de croissance (estimée par les différentes tailles des tamis), et leurs niveaux de ploïdie (vérifiés par cytométrie en flux).

De cette manière, des populations à fort développement dites « tête de lot », à développement moyen dites « corps de lot » et à développement lent dites « queue de lot » ont été constituées en fonction de ces deux critères, et mises en élevage dans des cuves séparées. Cette méthode de tri sélectif en fonction de la taille et de la ploïdie a pour but final de favoriser au plus la croissance et la survie des larves tétraploïdes et ceci en maximisant, au sein des différentes parties du lot d'élevage, leur effectif relativement aux larves diploïdes et triploïdes.

* Ainsi, à J8 post-fécondation, le premier tamisage sélectif sur la taille des larves a été réalisé et deux populations de larves ont été élevées séparément : la population « tête de lot I » ayant une croissance normale et une taille minimale de 80 µm et une population « queue de lot I » avec une croissance plus lente, et une taille comprise entre 65 µm et 80 µm.

Les tamisages suivants ont été effectués à J11, J13, J15, J18, J20, J23, J25, J27, et J29 post-fécondation.

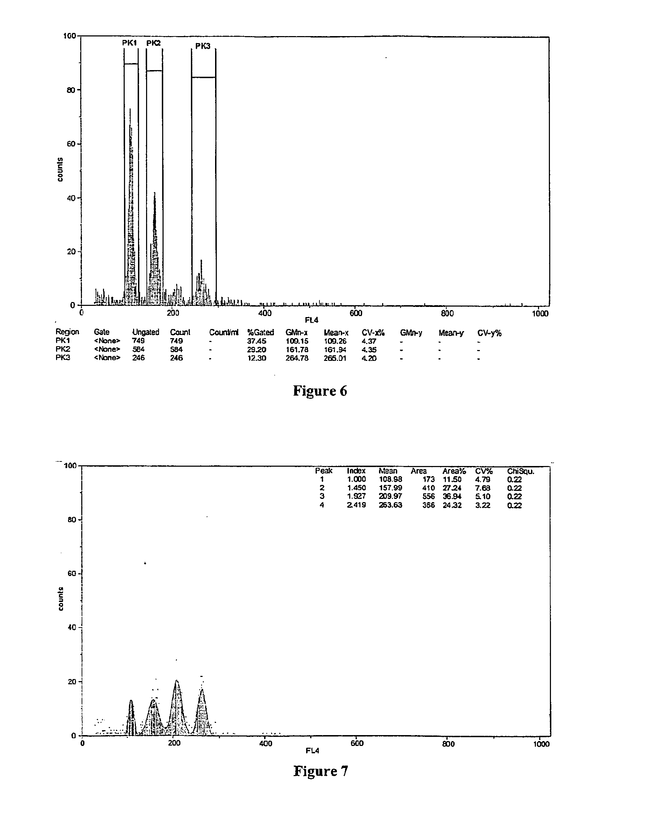

* A J11 post-fécondation, les analyses cytométriques réalisées sur le groupe témoin, ainsi que sur les deux populations de larves traitées à la CB montrent que :
- Le croisement témoin présente toujours une population de noyaux exclusivement diploïde (Figure 5, « peak 1 », le pic « peak 2 » correspondant au témoin interne TRBC),
- Les échantillons de larves traitées issues de la population « tête de lot I » ayant une taille minimale de 80 µm (T≥80µm) montrent que cette population est constituée uniquement de larves diploïdes (à 57%, « PK1 ») et de larves triploïdes (à 43%, « PK 2 »). Aucune proportion de larves tétraploïdes n'y a été détectée (Figure 6, le pic « PK 3 » correspondant au témoin interne TRBC).
- Les échantillons de larves traitées issues de la population « queue de lot I » ayant une taille inférieure à 80 µm (65µm <T< 80µm) montrent que cette population présente une constitution radicalement différente, que ce soit en termes de classes de ploïdie ou en terme de proportion de ces classes de ploïdie. Les analyses cytométriques montrent que cette population est constituée de 15% de larves diploïdes (peak 1), 40% de larves triploïdes (peak 2) et surtout de 45% de larves tétraploïdes (peak 3) (Figure 7 où le pic « peak 4 » correspond au témoin interne TRBC).

Ce résultat montre que la fraction tétraploïde de la population totale de larves en élevage est confinée dans la partie « queue de lot » de l'élevage larvaire confirmant ainsi notre hypothèse selon laquelle il existe un développement différentiel des larves ayant différents niveaux de ploïdie lorsqu'elles sont élevées en mélange. Ainsi, le niveau de ploïdie tétraploïde se révèle comme étant celui qui confère le moins de vitesse de croissance lorsqu'il est en compétition avec les niveaux diploïdes et triploïdes.

Par la suite de l'élevage, un soin particulier a été accordé à la population « queue de lot I », la seule contenant des tétraploïdes, et deux tamisages sélectifs ont été réalisés sur cette même population « queue de lot I » afin de pouvoir isoler deux sous-populations de plus en plus riches en larves tétraploïdes et ceci après tamisage au travers des tamis 60 et 80 µm.

Ainsi, la première sous population (appelée « queue de lot I ») est constituée des larves dont la taille est supérieure à 65 µm, et inférieure à 80µm, alors que la deuxième sous-population (appelée « corps de lot I ») est constituée des larves dont la taille est supérieure ou égale à 80 µm, et va généralement jusqu'à 100µm.

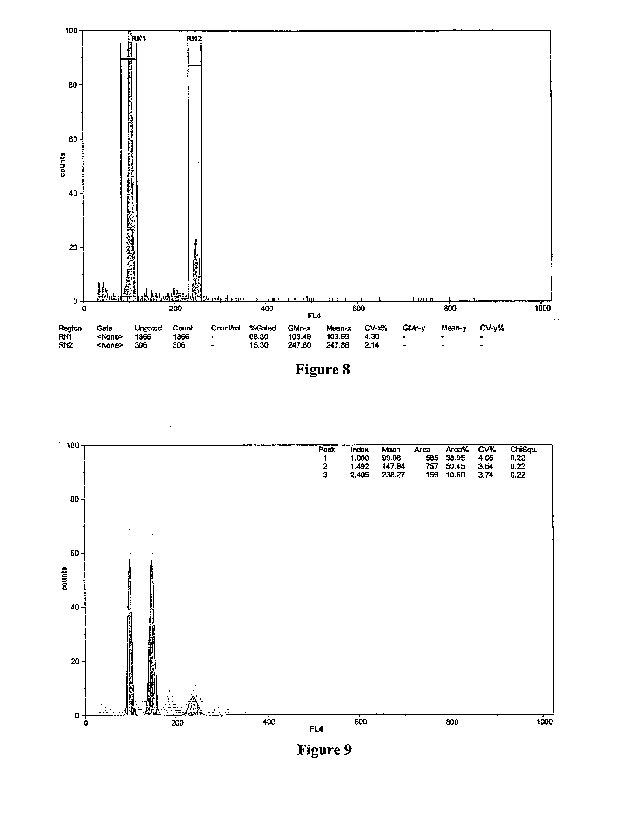

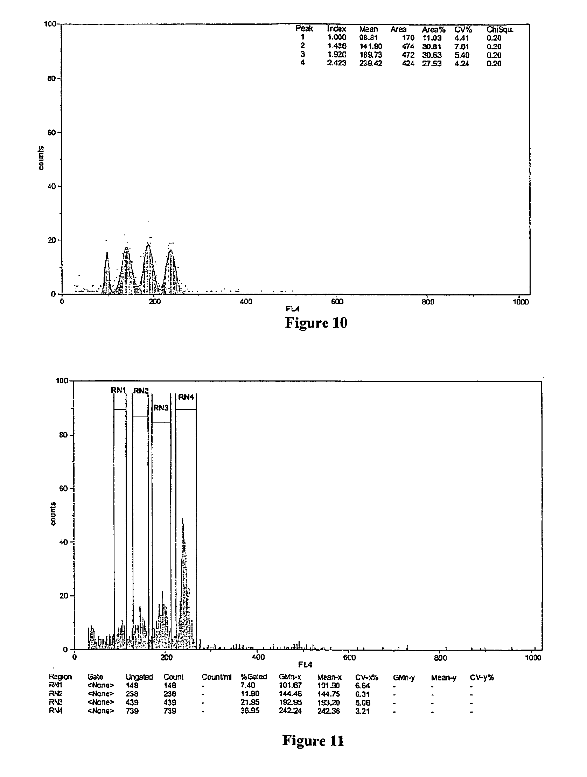

* A J13 post-fécondation, les analyses cytométriques réalisées à partir de larves traitées ou non à la CB montrent que :
- Le témoin non traité continue à ne montrer que des noyaux exclusivement diploïdes (pic « RN 1 » de la Figure 8, le pic « RN 2 » correspondant au témoin interne TRBC).
- La population des larves traitées « tête de lot I » continue à être exclusivement constituée de larves diploïdes (45%, peak1) et triploïdes (55%, peak2), (Figure 9 où le « peak3 » correspond au témoin interne TRBC). Cette population est composée de larves qui présentent une croissance normale et qui ont atteint une taille minimale de 100 µm.
- Les populations des larves traitées « corps de lot I » et « queue de lot I » sont les deux seules qui continuent à montrer une forte proportion de larves tétraploïdes (40 à 50% représentés par les pics «peak 3» de la figure 10 et «RN 3» de la Figure 11). Les pics « peak 1-2-4 » et « RN 1-2-4 » correspondent respectivement aux noyaux diploïdes, triploïdes et témoin interne TRBC des figures 10 et 11).

* A J15 post-fécondation, nos efforts se sont concentrés sur la conduite de l'élevage des populations « corps de lot I » et « queue de lot I » des larves traitées (celles qui contiennent des tétraploïdes). Des tamisages sélectifs sont réalisés sur ces populations, en utilisant les tamis de 65, 100 et 125 µm de taille de mailles, afin de constituer de nouvelles sous-populations :
- à forte croissance dite « tête de lot II » avec une taille comprise entre 125 et 150 µm ;
- à croissance moyenne dite « corps de lot II » avec une taille comprise entre 100 et 125 µm ;
- à faible croissance dite « queue de lot II » avec une taille comprise entre 65 et 100 µm.

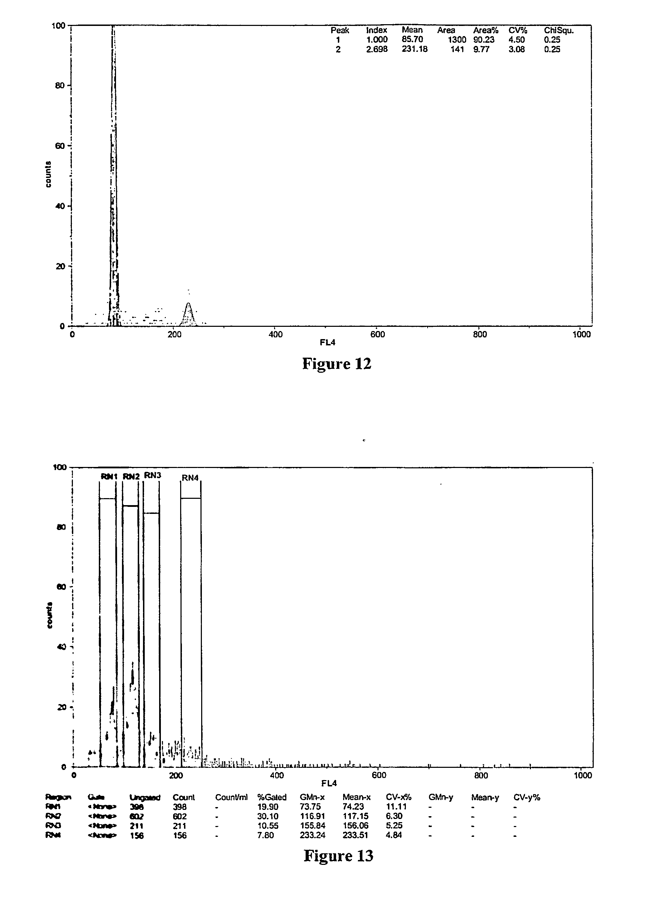

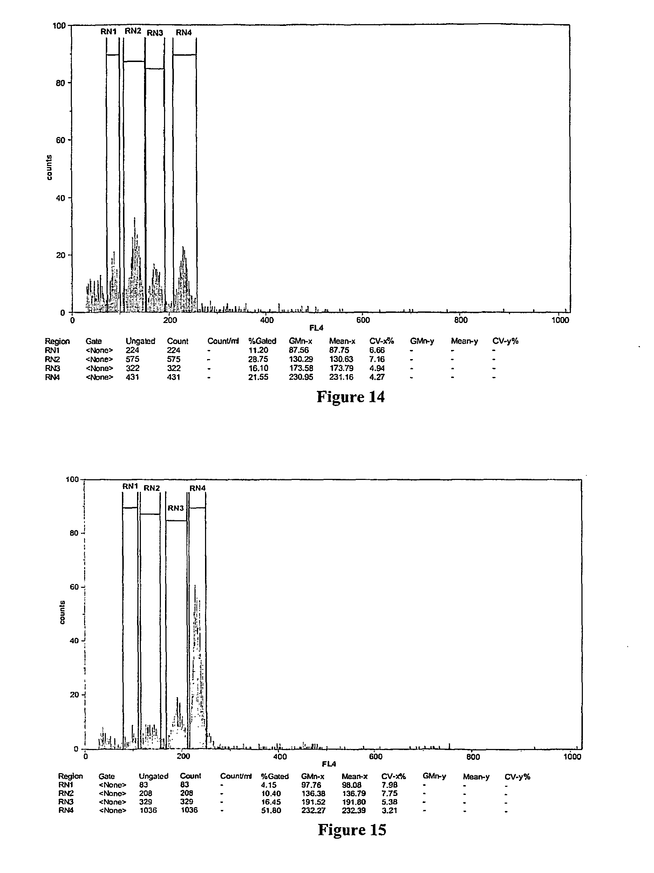

Les analyses cytométriques réalisées à partir de larves traitées ou non à la CB montrent que :
- Le témoin non traité continue à ne montrer que des noyaux exclusivement diploïdes (« peak1 », Figure 12 ; le « peak 2 » correspond au témoin interne TRBC).
- La population des larves traitées « tête de lot I » reste constituée exclusivement de larves diploïdes (à hauteur de 45%) et triploïdes (à hauteur de 55%).
- Les populations des larves traitées à la CB « tête de lot II » (150µm >T≥ 125µm), « corps de lot II » (125µm >T≥ 100µm) et « queue de lot II » (100µm >T≥ 65µm) continuent de montrer une proportion notable de larves tétraploïdes (Figures 13, 14, 15 où les pics « RN 1-2-3-4 » correspondent respectivement aux noyaux diploïdes, triploïdes, tétraploïdes, et témoin interne TRBC). Toutefois, l'importance de cette population tétraploïde est très différente d'une partie du lot d'élevage à l'autre. Cette proportion de larves tétraploïdes est la plus basse (15%) dans la « tête de lot II » (Figure 13, pic « RN3 »), et la plus haute (50%) dans la « queue de lot II » (Figure 15, pic « RN3 »). Dans la partie « corps de lot II », cette proportion de larves tétraploïdes présente une valeur intermédiaire (28%, Figure 14, pic « RN3 »). II apparaît donc clairement que les lots d'élevage présentant le plus de larves tétraploïdes sont ceux qui croissent le moins vite ce qui souligne encore une fois de plus la différence de valeur d'adaptation générale des trois niveaux de ploïdie et la nécessité de réalisation de tris sélectifs afin de maintenir les larves tétraploïdes.

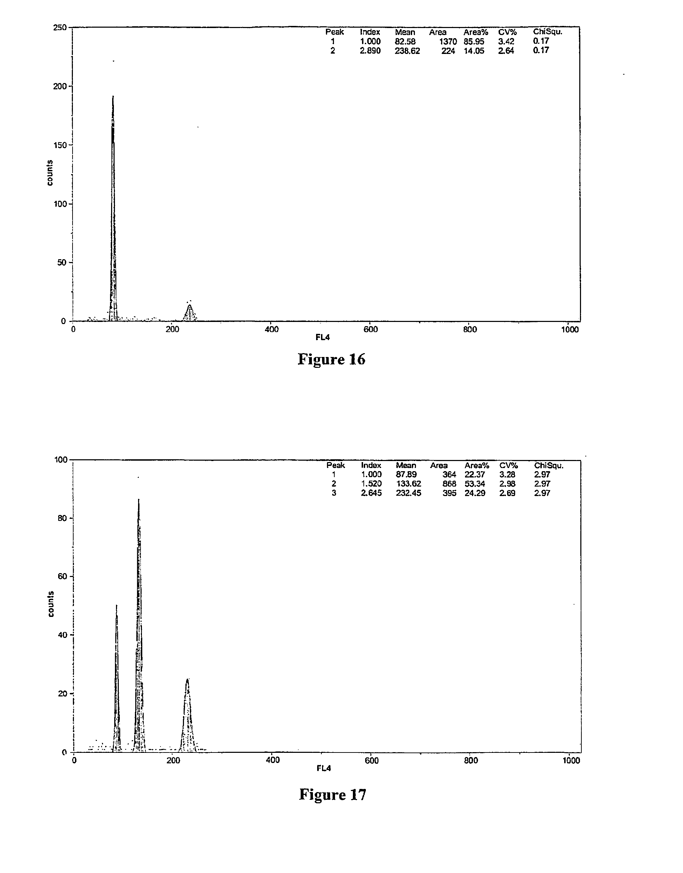

* A J18 post-fécondation, une partie des larves traitées de la population « tête de lot I » (T ≥ 250µm) sont pédivéligères et commencent à se fixer et ceci deux jours avant les larves témoins non traitées. Les analyses cytométriques réalisées à J18 continuent à montrer un niveau exclusivement diploïde chez le témoin (Figure 16, « peak1 »; le « peak2 » correspond au témoin interne TRBC), et un mélange de diploïdes (à 30%, « peak1 ») et de triploïdes (à 70 %, peak2 ») chez la population « tête de lot I » (Figure 17 où le « peak 3 » correspond au témoin interne TRBC).

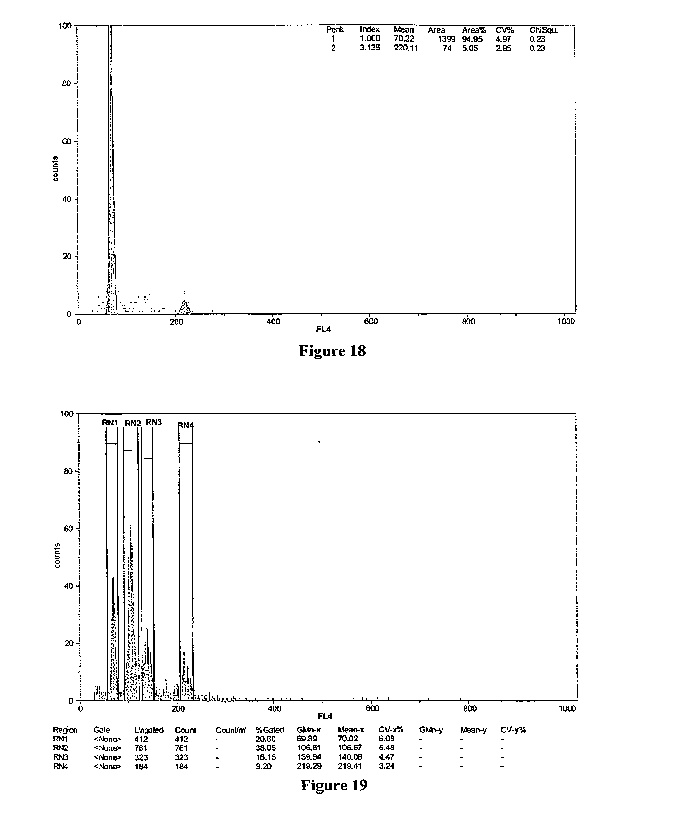

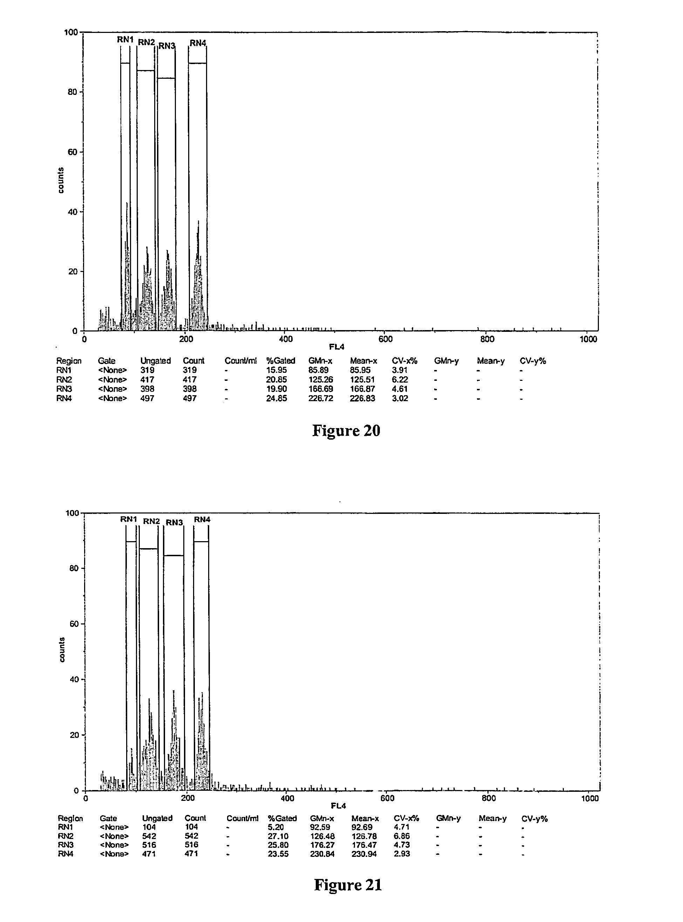

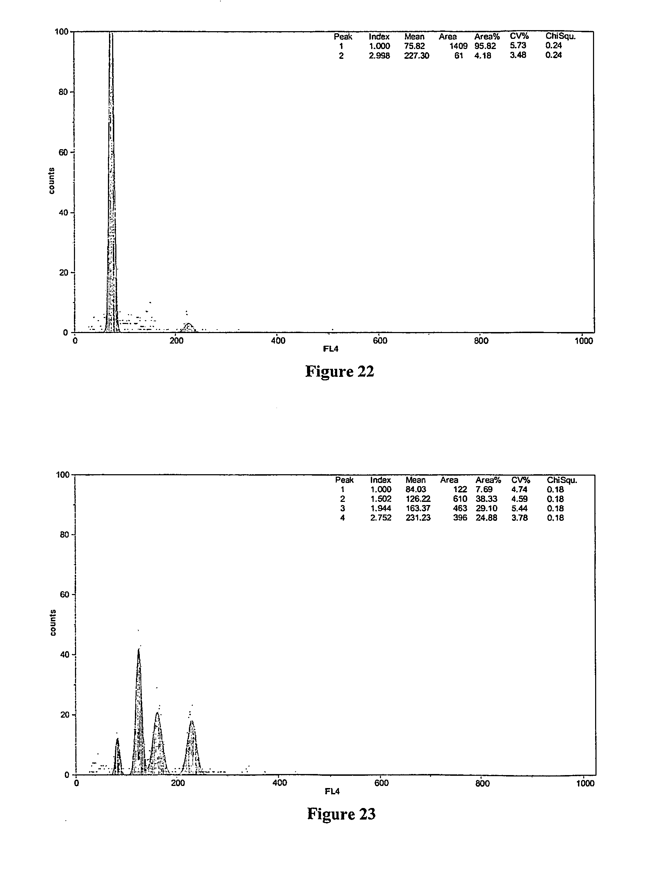

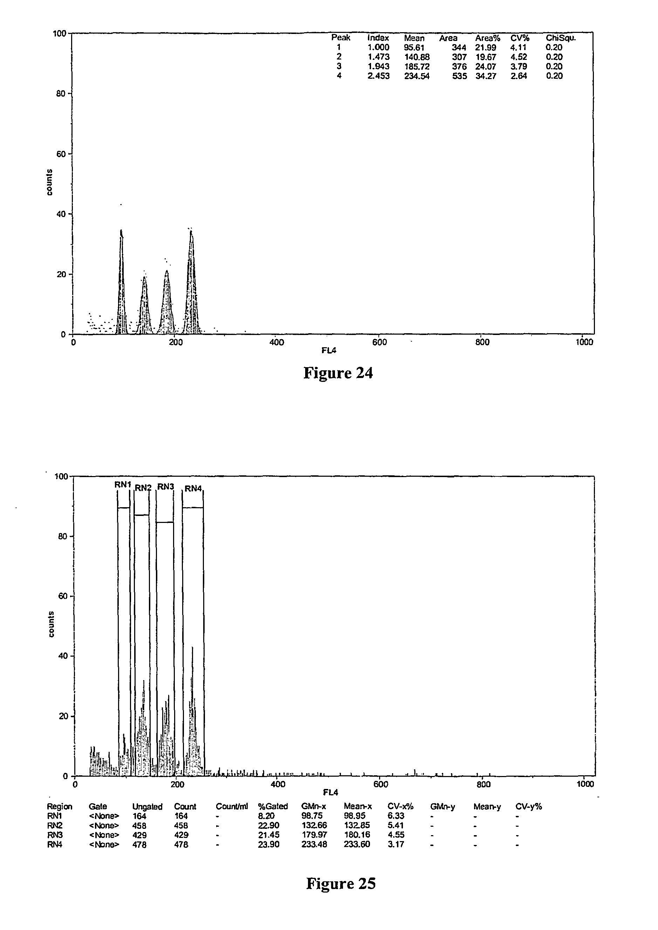

* A J20 et J23 post-fécondation, tandis que le témoin non traité continue à montrer un niveau exclusivement diploïde (Figures 18 et 22, « peak1 »), les lots d'élevage contenant des larves tétraploïdes (tête de lot II (T ≥ 180 µm) ; corps de lot II (180 µm > T ≥ 150µm) et queue de lot II (150µm > T ≥ 100µm)) montrent sensiblement les mêmes proportions de larves tétraploïdes qu'à J15 post-fécondation (respectivement 20, 35 et 45% de tétraploïdes représentés par les pics « RN/peak 3 » des Figures 19-21 et 23-25, où les autres pics « peak/RN 1-2-4 » correspondent respectivement aux diploïdes, triploïdes, et témoin interne TRBC). De plus, même si leur croissance parait manifestement plus lente que celle du témoin non traité, ces larves ont malgré tout vu leur taille minimale augmenter (tailles minimales respectives de 180, 150 et 100 µm à J23 contre 125, 100 et 65 µm à J15 post-fécondation).

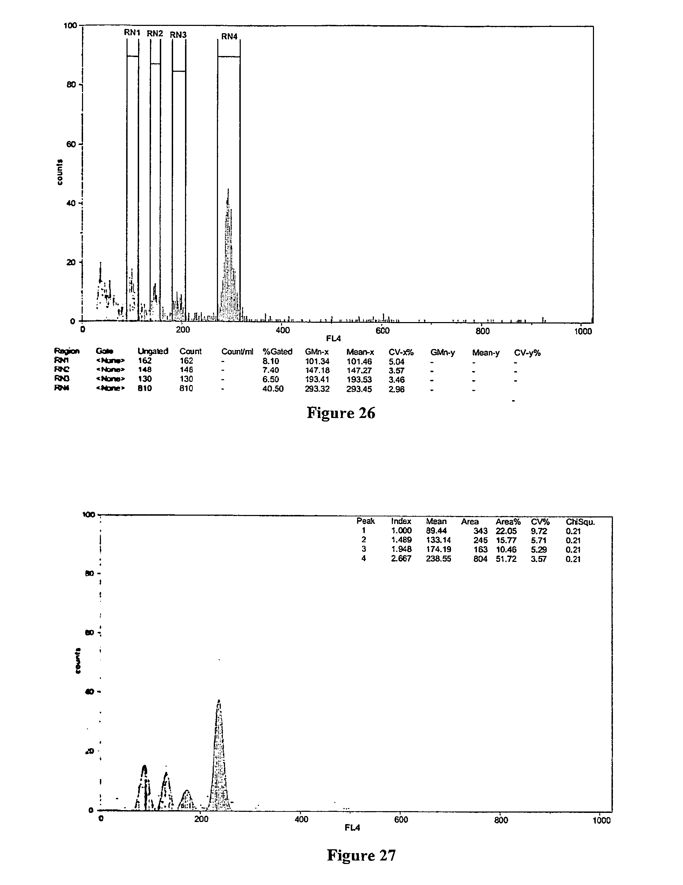

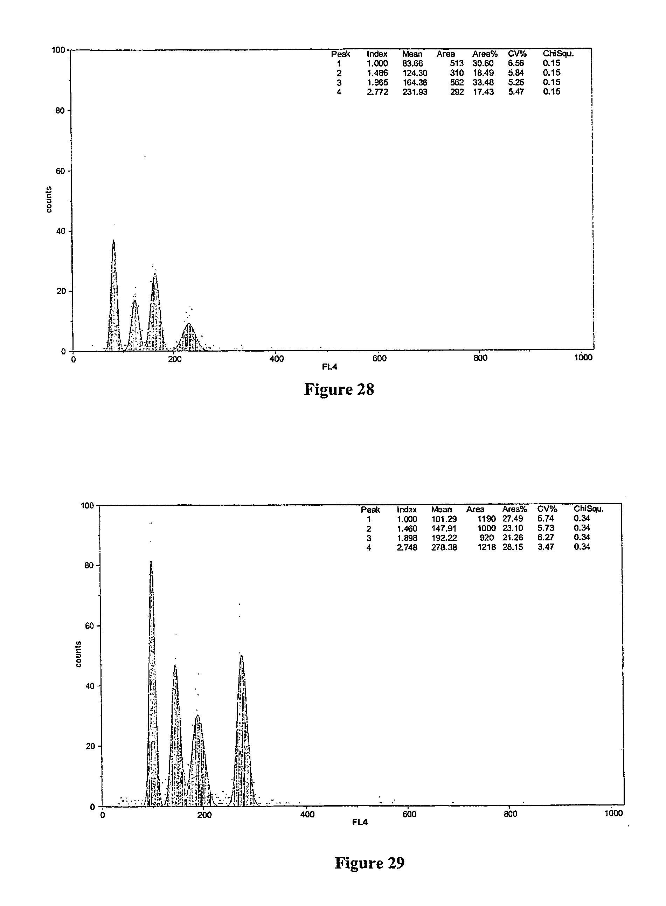

* A J25 post-fécondation, une première cohorte de larves pédivéligères (T ≥ 250µm) contenant 30% de larves tétraploïdes (pic « RN3 » de la Figure 26, les autres pics « RN 1-2-4 » correspondant respectivement aux diploïdes, triploïdes, et au témoin interne TRBC)) a été mise en fixation sur de la microbrisure de coquilles d'huîtres. Cette première fixation de larves tétraploïdes a été suivie à J27 et J29 de trois autres mises en fixations de larves pédivéligères contenant des proportions variables de larves tétraploïdes allant de 20 à 40% (pics « peak3 » des Figures 27, 28 et 29. les autres pics « peak 1-2-4 » correspondent respectivement aux noyaux diploïdes, triploïdes, et témoin interne TRBC).

### EXEMPLE 2 : FIXATION ET DEVELOPPEMENT DE NAISSAINS TETRAPLOÏDES

### 1 mois post-fixation

Le contrôle de la présence de naissains tétraploïdes au sein de la population de larves qui a réussi à se métamorphoser et à se fixer a été réalisé un mois post-fixation, par cytométrie de flux. A cause de la petite taille du naissain à ce stade, ce contrôle a été réalisé en mode destructif sur un échantillon de plusieurs naissains à la fois. Les naissains sont mis dans un tube de 1,5 ml. Ensuite, ils sont incubés dans 1 ml de tampon de lyse et broyés à l'aide d'un piston en plastique. Enfin, les noyaux libérés sont filtrés au travers d'un filtre en nylon de 30 µm de taille de maille (Partec Celltrics) et recueillis dans un tube d'analyse de cytométrie, marqués au DAPI, et analysés comme décrit à l'Exemple 1 ci dessus.

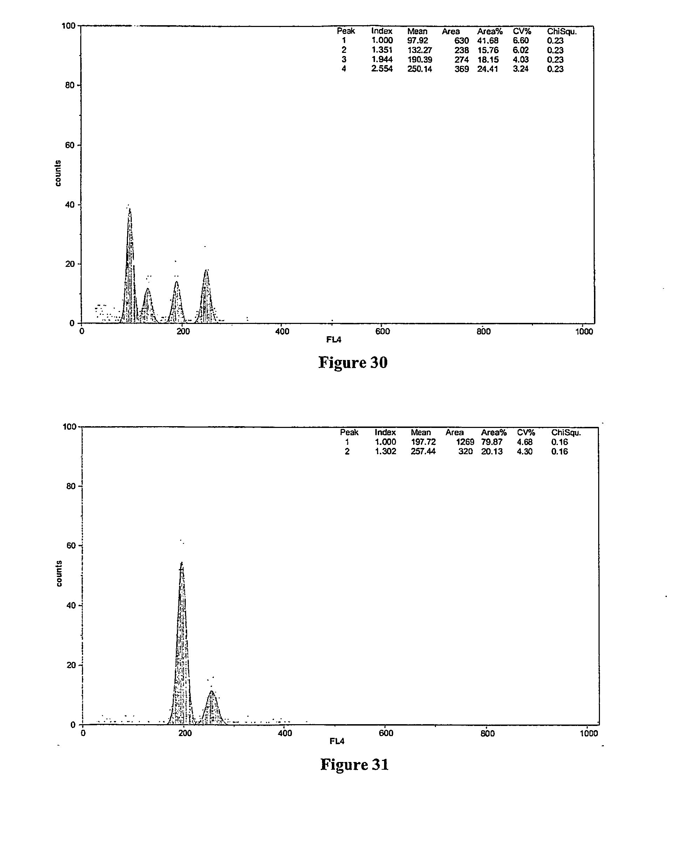

Les résultats cytométriques nous ont permis de confirmer la présence, à côté de populations triploïdes et diploïdes, d'une population notable de naissains tétraploïdes (29%) (« peak3 » de la Figure 30 ; les pics «peak1-2-4» correspondent respectivement aux noyaux diploïdes, triploïdes et témoin interne TRBC). Cette population de naissains tétraploïdes a donc réussi à se fixer et à poursuivre son développement en micronursage.

Ce résultat démontre l'obtention, directement à partir de géniteurs diploïdes, de naissain tétraploïde ayant survécu à la phase d'élevage larvaire et à la métamorphose permettant la fixation. De plus le naissain tétraploïde obtenu continue à se développer même après fixation.

### 2 mois post-fixation

Deux mois après fixation, le contrôle de la présence des naissains tétraploïdes au sein de la population de naissains âgés de trois mois a été réalisé, toujours par cytométrie en flux, en mode destructif mais sur des naissains individualisés.

Nos résultats nous ont permis de montrer la présence de naissains tétraploïdes âgés de trois mois (Figure 31). Comme démontré durant l'élevage larvaire, la mise en relation durant l'élevage en micronurserie, du niveau de ploïdie d'un individu avec sa taille révèle aussi un développement différentiel. En effet, la population de naissains tétraploïdes ayant une croissance lente apparaît se cantonner plutôt dans les parties « queue de lot » et « corps de lot » de l'élevage alors que la partie « tête de lot » apparaît comme étant constituée de naissains diploïdes et triploïdes se développant plus rapidement.

Pour la suite, et dans le but de favoriser la croissance des tétraploïdes, nous avons donc adopté la même démarche que celle adoptée durant l'élevage larvaire : à savoir un système de tri des naissains en fonction de leur taille et de leur niveau de ploïdie de façon à constituer des lots d'élevage avec le plus possible de naissains tétraploïdes et subissant le moins possible de concurrence de la part des naissains triploïdes et diploïdes.

Pratiquement, les naissains individualisés ont subi des tamisages sélectifs successifs au travers de tamis rectangulaires (45x35x12 cm) dont la taille de l'ouverture des mailles s'échelonnait de 150µm à 2 mm en passant par les tailles intermédiaires de 350, 500 et 1000 µm.

Ces tamisages sélectifs ont été effectués à intervalles réguliers de 15 jours. Selon une démarche similaire à celle suivie lors de l'étape de l'élevage larvaire, des populations « tête de lot », « corps de lot » et « queue de lot » ont été constituées et élevées séparément.

### 6 mois post-fixation

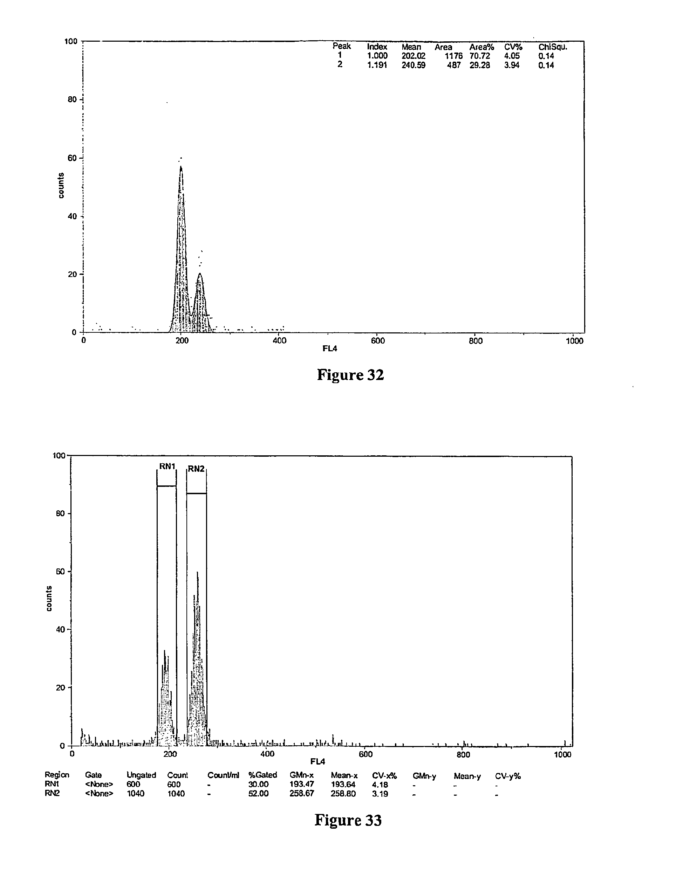

A six mois post-fixation, les naissains âgés de 7 mois commencent à être accessibles à un marquage individuel et à un contrôle non destructif de leur niveau de ploïdie à partir de biopsies branchiales. Ainsi, chaque individu a été identifié par une étiquette en plastique collée à la colle epoxy sur sa coquille. Ensuite, les individus à analyser sont anesthésiés dans un bain de 3 litres d'eau de mer contenant 150 g de MgCl₂, permettant ainsi le prélèvement de la biopsie branchiale sur laquelle sera réalisée la mesure de la ploïdie de l'animal. A ce stade, les analyses cytométriques réalisées sur un total de 600 individus nous ont permis de trier plus de 90 jeunes huîtres tétraploïdes avérées soit un taux moyen de 15% d'huîtres tétraploïdes directement induites à partir de géniteurs diploïdes sauvages (Figure 32).

### 8-9 mois post-fixation

A huit mois post-fixation, nos analyses cytométriques nous permis de réaliser un tri de plus de 200 individus tétraploïdes trouvés surtout dans les populations « queue de lot » et « corps de lot ».

Pour des raisons de places disponibles, le tri des individus a été arrêté à ce stade.

A neuf mois post-fixation, une vingtaine d'individus dont la taille moyenne était de 6 cm ont été mis en conditionnement permettant le déclenchement de la maturation et la production de gamètes. Ces futurs géniteurs ont été conditionnés dans une eau de mer à 20°C, contenant 2.10⁹ cellules de phytoplancton par jour et par individu. Au terme de cette période de conditionnement à la maturation, les géniteurs tétraploïdes ont été soumis à plusieurs chocs (thermiques et d'exondation) afin de déclencher leur ponte. Sur la trentaine de géniteurs mis en maturation, 18 ont pondu avec un sex ratio nettement favorable aux mâles (17 mâles et 1 femelle) ce qui est parfaitement conforme à la nature protandre de l'huître creuse *C*. *gigas.* Afin de tester les gamètes produits par ces géniteurs tétraploïdes, nous avons réalisé deux types de croisements :

Le premier en fécondant les ovocytes tétraploïdes, issus de la seule femelle tétraploïde, par le mélange du sperme tétraploïde, produit par les 17 mâles tétraploïdes.

Le second en fécondant des ovocytes issus de six femelles diploïdes sauvages par le sperme issu des 17 mâles tétraploïdes.

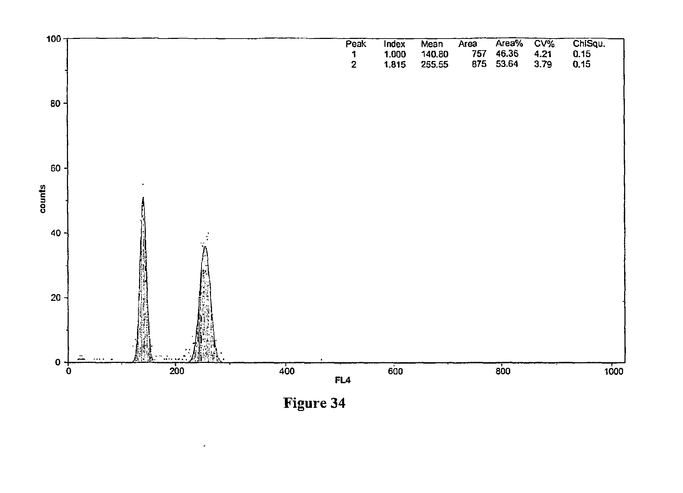

Nos résultats montrent clairement que les gamètes produits par les géniteurs testés sont pleinement viables et fécondants. Ainsi, croisés entre eux, les nouveaux tétraploïdes produisent des larves uniquement tétraploïdes (Figure 33, 100% de tétraploïdes représentés par le pic « RN1 », le pic « RN2 » représentant le témoin interne TRBC), et croisés avec des femelles diploïdes, les mâles tétraploïdes produisent une descendance uniquement triploïde (Figure 34, 100% de triploïdes représentés par le pic « peak1 », le pic « peak2 » représentant le témoin interne TRBC). Actuellement, ces nouvelles générations de tétraploïdes et de triploïdes se sont métamorphosées et fixées et sont en élevage en micronurserie (taille entre 500 et 1000 µm).

En conclusion, nous avons réussi à produire des huîtres tétraploïdes issues de géniteurs diploïdes. Ces mêmes tétraploïdes, après un traitement de conditionnement de la maturation gamétique, réussissent à entrer en gamétogenèse et à produire des gamètes mâles et femelles tétraploïdes pleinement viables et fécondants. Quand ces tétraploïdes ont été croisés entre eux, ils réussissent à produire une nouvelle génération de tétraploïdes pleinement viables. Enfin, quand ils ont été utilisés comme géniteurs mâles servant à féconder des femelles diploïdes, ces même tétraploïdes produisent une nouvelle génération de triploïdes aussi pleinement viables ce qui ouvre ainsi la voie à l'utilisation de ces tétraploïdes comme possibles géniteurs fournissant la filière triploïde.

### Au-delà de 9 mois post fixation (février 2007 - mars 2008)

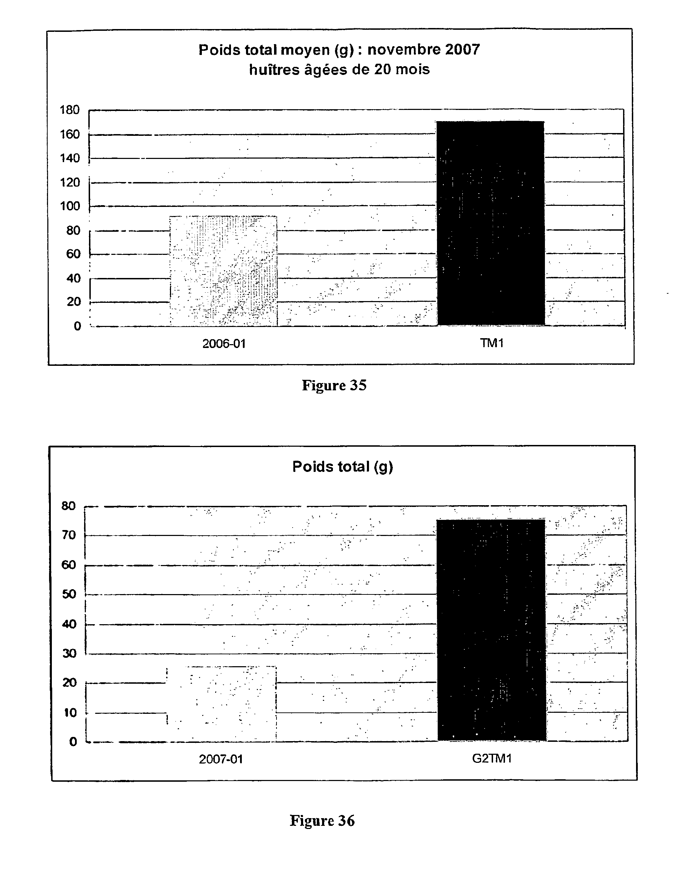

Le niveau de ploïdie des huîtres tétraploïdes de première génération issues du croisement de mâles et de femelles diploïdes (huîtres tétraploïdes directes dénommées ci-après « TM1 ») est confirmé par analyses cytométriques et aussi après comptage chromosomique à partir de tissu branchial en utilisant les techniques de caryotype classiques. Comparativement aux huîtres tétraploïdes classiques (c'est à dire celles issues de femelles triploïdes) de même âge et élevées dans les mêmes conditions, les huîtres tétraploïdes directes « TM1 » de première génération ont montré une grande rusticité et une croissance pondérale bien plus importantes (Figure 35). Ainsi, au bout de vingt mois d'élevage, les tétraploïdes de première génération (TM1) sont 1,86 fois plus lourdes que les tétraploïdes classiques (2006-01). De plus le cheptel TM1 s'est montré particulièrement résistant puisque aucune mortalité ne s'y est manifestée à la différence du cheptel de tétraploïdes classiques (2006-01) qui a connu plusieurs épisodes de mortalité surtout estivale.

Début 2007, les huîtres tétraploïdes directes de première génération « TM1 » ont été utilisées comme géniteurs mâles et femelles afin de produire, suite à une ponte naturelle, une deuxième génération d'huîtres tétraploïdes directes dénommée ci-après « G2TM1 ». La tétraploïdie des larves et naissains G2TM1 a été aussi confirmée à la fois par analyses cytométriques et aussi après comptage chromosomique en utilisant les techniques de caryotype classiques. Les naissains G2TM1 sont tous tétraploïdes et leur élevage, que ce soit au stade larvaire, micronurserie ou nurserie, ne diffère aucunement d'un élevage classique. Durant leur élevage, les naissains G2TM1 ont montré un très bon comportement surtout en termes de rusticité, de croissance et de stabilité du niveau de tétraploïdie. En effet, comparativement aux naissains tétraploïdes (2007-01) classiques (c'est à dire issus de femelles triploïdes) de même âge et élevés dans les mêmes conditions, et même si leur niveau de réversion vers les niveaux de ploïdie inférieurs ne semble pas différent de celui présenté par les naissains tétraploïdes classiques, les naissains G2TM1 présentent une croissance et une rusticité bien supérieures. En effet, au bout de 9 mois d'élevage, le cheptel G2TM1 se compose d'individus tétraploïdes dont le poids total moyen oscille autour 75 grammes alors que celui des tétraploïdes classiques était seulement autour de 27 grammes ce qui veut dire que les individus G2TM1 étaient en moyenne 2.7 fois plus lourds que les tétraploïdes classiques de même âge (Figure 36). De plus, le cheptel G2TM1 s'est montré particulièrement rustique puisque aucune mortalité n'y est apparue contrairement au cheptel 2007-01.

A partir du mois de novembre 2007, les tétraploïdes G2TM1 âgés de 9 mois post fixation ont été conditionnés à la maturation, et deux mois et demi plus tard, utilisés comme géniteurs afin de produire, par ponte induite, des gamètes qui ont été utilisés afin de produire en février 2008 la troisième génération de tétraploïdes directs dénommée ci-après « G3TM1 ». Le sex ratio des géniteurs tétraploïdes G2TM1 ainsi que leur fécondité ne différent pas de ceux des géniteurs diploïdes. Les larves G3TM1 produites sont bien tétraploïdes. Parallèlement, Les mâles G2TM1 ont aussi été efficacement utilisés afin de féconder des ovocytes produits par des femelles diploïdes et ceci dans le but de produire des triploïdes.

Les résultats ci-dessus montrent que les tétraploïdes obtenus conformément à l'invention sont très efficaces afin de produire des triploïdes. De même que chez les diploïdes, les huîtres tétraploïdes arrivent à maturité au cours de leur première année d'âge. Ces huîtres (Crassostrea gigas Thunberg) tétraploïdes matures présentent un sex-ratio normal et une fécondité comparable à celle des diploïdes. Les croisements entre tétraploïdes et diploïdes sont facilement et normalement réalisés. Tous les croisements tétraploïdes x diploïdes (et réciproquement) produisent des naissains exclusivement triploïdes après contrôles de la ploïdie par cyométrie en flux.

### EXEMPLE 3 : OBTENTION DE TETRAPLOÏDES CHEZ LA MOULE

Des tétraploïdes ont été efficacement obtenus chez la moule (*M. edulis* et *Mytilus galloprovincialis*) en utilisant la procédure détaillée dans les exemples 1 et 2.

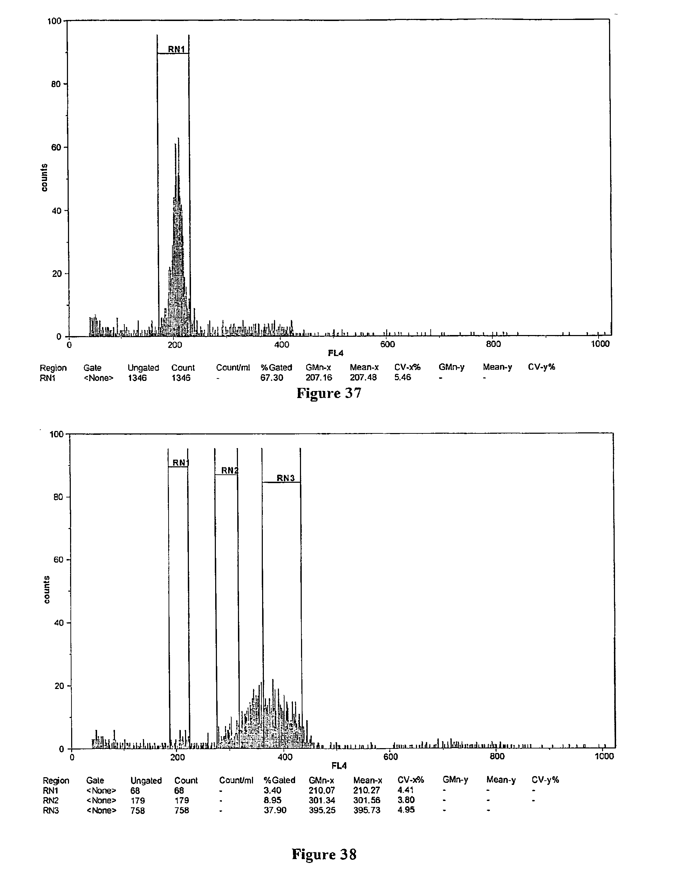

Chez la moule, les résultats cytométriques nous ont permis de confirmer la présence, à côté de populations triploïdes et diploïdes, d'une population majoritaire de naissains tétraploïdes. Ainsi,
- Le croisement témoin présente une population de noyaux exclusivement diploïdes (Figure 37, pic « RN 1 »),
- Les échantillons issus de l'induction de rétention de GPI montrent trois types de populations nucléaires (Figure 38) correspondant aux niveaux de ploïdie suivants: diploïde (autour de 10% en moyenne, pic « RN 1 »), triploïde (autour de 20 % en moyenne, pic « RN 2 ») et tétraploïde (autour de 70% en moyenne, pic « RN 3 »). Les individus tétraploïdes obtenus sont en cours d'élevage.

D'une manière générale et de la même façon que chez l'huître creuse et la moule, des tétraploïdes viables peuvent être produits chez tous les mollusques bivalves en utilisant la procédure détaillée dans les exemples ci-dessus.

## Revendications

1. Procédé d'obtention de mollusques bivalves tétraploïdes, **caractérisé en ce qu'**il comprend :
a) la fécondation d'ovocytes de femelles diploïdes par du sperme de males diploïdes, suivie de l'induction de la rétention du premier corps polaire des oeufs fécondés ;
b) la culture des larves obtenues à l'issue de cette fécondation ;
c) l'isolement, à partir de la population de larves cultivées à l'étape b), d'une sous-population enrichie en tétraploïdes, comprenant au moins 20% de larves tétraploïdes ;
d) la culture de la sous-population de larves isolée à l'étape c).

2. Procédé selon la revendication 1, **caractérisé en ce que** la rétention du premier corps polaire est induite par un traitement à la cytochalasine B, effectué entre la 5^{ème} et la 25^{ème} minute après la fécondation, pendant 10 à 20 minutes.

3. Procédé selon la revendication 2, **caractérisé en ce que** la cytochalasine B est utilisée à une concentration finale par litre de milieu de traitement comprise entre 0,3 et 0,7 mg.

4. Procédé selon une quelconque des revendications 1 à 3, **caractérisé en ce que** la culture de l'étape b) est effectuée pendant 6 à 8 jours.

5. Procédé selon une quelconque des revendications 1 à 4, **caractérisé en ce que** la culture de l'étape d) est effectuée pendant 2 à 3 jours.

6. Procédé selon une quelconque des revendications 1 à 5, **caractérisé en ce qu'**il comprend les étapes supplémentaires suivantes :
e) l'isolement, à partir de la sous-population de larves cultivées à l'étape précédente, d'une sous-population riche en tétraploïdes, comprenant au moins 20% de larves tétraploïdes ;
f) la culture de la sous-population de larves isolée à l'étape e).

7. Procédé selon la revendication 6, **caractérisé en ce que** la culture de l'étape f) est effectuée pendant 2 à 3 jours.

8. Procédé selon une quelconque des revendications 1 à 7, **caractérisé en ce que** l'isolement des sous-populations enrichies en tétraploïdes est effectué en triant les larves sur la base de leur taille.

9. Procédé selon la revendication 8, **caractérisé en ce que** l'isolement des sous-populations enrichies en tétraploïdes est effectué par passage sur un tamis de taille de maille appropriée, et sélection des larves qui ne sont pas retenues par le tamis.

10. Procédé selon une quelconque des revendications 1 à 9, **caractérisé en ce que** lesdits mollusques bivalves sont des huîtres.

11. Procédé selon une quelconque des revendications 1 à 9, **caractérisé en ce que** lesdits mollusques bivalves sont des moules.

## Claims

1. Process for the production of bivalve tetraploid molluscs, **characterized in that** it comprises:
a) fertilization of ovocytes from diploid females with sperm from diploid males, followed by induction of the retention of the first polar body of the fertilized eggs;
b) cultivation of the larvae obtained after this fertilization;
c) isolation, from the population of larvae cultivated in step b), of a tetraploid-enriched subpopulation comprising at least 20% of tetraploid larvae; and
d) cultivation of the subpopulation of larvae isolated in step c).

2. Process according to Claim 1, **characterized in that** the retention of the first polar body is induced by a 10-to 20-minute treatment with cytochalasine B carried out between 5 and 25 minutes after fertilization.

3. Process according to Claim 2, **characterized in that** the cytochalasine B is used at a final concentration of between 0.3 and 0.7 mg per litre of treatment medium.

4. Process according to any one of Claims 1 to 3, **characterized in that** the cultivation of step b) is carried out for 6 to 8 days.

5. Process according to any one of Claims 1 to 4, **characterized in that** the cultivation of step d) is carried out for 2 to 3 days.

6. Process according to any one of Claims 1 to 5, **characterized in that** it comprises the following additional steps:
e) isolation, from the subpopulation of larvae cultivated in the previous step, of a tetraploid-rich subpopulation comprising at least 20% of tetraploid larvae; and
f) cultivation of the subpopulation of larvae isolated in step e).

7. Process according to Claim 6, **characterized in that** the cultivation of step f) is carried out for 2 to 3 days.

8. Process according to any one of Claims 1 to 7, **characterized in that** the isolation of the tetraploid-enriched subpopulations is carried out by sorting the larvae on the basis of their size.

9. Process according to Claim 8, **characterized in that** the isolation of the tetraploid-enriched subpopulations is carried out by passage through a sieve of appropriate mesh size and selection of the larvae not retained by the sieve.

10. Process according to any one of Claims 1 to 9, **characterized in that** said bivalve molluscs are oysters.

11. Process according to any one of Claims 1 to 9, **characterized in that** said bivalve molluscs are mussels.

## Patentansprüche

1. Verfahren zur Gewinnung von tetraploiden zweischaligen Mollusken, **dadurch gekennzeichnet , dass** es umfasst:
a) die Befruchtung von Oozyten von diploiden Weibchen durch das Sperma von diploiden Männchen, gefolgt von der Induzierung der Retention des ersten polaren Körpers der befruchteten Eier;
b) die Kultur der Larven, die aus dieser Befruchtung erhalten wurden;
c) Isolierung, aus der Population der kultivierten Larven in Stufe b), einer Subpopulation, die an tetraploiden angereichert ist, die wenigstens 20% tetraploide Larven umfasst;
d) die Kultur der Unterpopulation von in Stufe c) isolierten Larven.

2. Verfahren gemäß Anspruch 1, **dadurch gekennzeich**n e t , dass die Retention des ersten polaren Körpers durch eine Behandlung mit Cytochalasin B induziert wird, die zwischen der 5. und der 25. Minute nach der Befruchtung während 10 bis 20 Minuten durchgeführt wird.

3. Verfahren gemäß Anspruch 2, **dadurch gekennzeichnet , dass** das Cytochalasin B in einer Endkonzentration von zwischen 0,3 und 0,7 mg pro Liter Behandlungsmedium verwendet wird.

4. Verfahren gemäß einem der Ansprüche 1 bis 3, **dadurch gekennzeichnet , dass** die Kultur der Stufe b) während 6 bis 8 Tagen durchgeführt wird.

5. Verfahren gemäß einem der Ansprüche 1 bis 4, **dadurch gekennzeichnet , dass** die Kultur der Stufe d) während 2 bis 3 Tagen durchgeführt wird.

6. Verfahren gemäß einem der Ansprüche 1 bis 5, **dadurch gekennzeichnet , dass** es die zusätzlichen folgenden Schritte umfasst:
e) die Isolierung einer Unterpopulation, die an Tetraploiden angereichert ist, die wenigstens 20% tetraploide Larven umfasst, ausgehend von der Unterpopulation der in der vorangehenden Stufe kultivierten Larven;
f) die Kultur der in Stufe e) isolierten Unterpopulation von Larven.

7. Verfahren gemäß Anspruch 6, **dadurch gekennzeich**n e t , dass die Kultur der Stufe f) während 2 bis 3 Tagen durchgeführt wird.

8. Verfahren gemäß einem der Ansprüche 1 bis 7, **dadurch gekennzeichnet , dass** die Isolierung der Unterpopulationen, die an Tetraploiden angereichert sind, durchgeführt wird, indem die Larven auf der Basis ihrer Größe sortiert werden.

9. Verfahren gemäß Anspruch 8, **dadurch gekennzeich** - **net**, dass die Isolierung der Unterpopulationen, die an Tetraploiden angereichert sind, durch Passage durch ein Sieb mit geeigneter Maschengröße und Selektion der Larven, die nicht durch das Sieb zurückgehalten werden, erfolgt.

10. Verfahren gemäß einem der Ansprüche 1 bis 9, **dadurch gekennzeichnet dass** die zweischaligen Mollusken Austern sind.

11. Verfahren gemäß einem der Ansprüche 1 bis 9, **dadurch gekennzeichnet , dass** die zweischaligen Mollusken Muscheln sind.
